# EUROPEAN PATENT APPLICATION

(11) **EP 1 188 837 A1**
(43) Date of publication of application: **20.03.2002**
(21) Application number: 00203230.8
(22) Date of filing: 18.09.2000
(51) Int. Cl.: C12P 17/10, C12P 17/12, C12N 1/21, C12N 9/02

(54) **Process for preparing N-substituted 4-hydroxypiperidines by enzymatic hydroxylation**

(71) Applicant: Eidgenössische Technische Hochschule Zürich, 8093 Zürich (CH)
(72) Inventor: Li, Zhi, 8902 Urdorf (CH); Witholt, Bernard, 8032 Zurich (CH); Chang, Dongliang, 8048 Zurich (CH)
(74) Representative: Prins, Adrianus Willem

(57) **Abstract**

A process for the preparation of N-substituted 4-hydroxypiperidine, wherein an oxygen atom is inserted regioselectively into the corresponding N-substituted piperidine, by using as a biocatalyst a bacterium degrading alkanes or alicyclic hydrocarbons, or a prokaryotic host-organism having the gene(s) necessary for the hydroxylation derived from the said bacterium, or an enzyme having hydroxylation activity derived therefrom. The bacterium may be selected from species from, for example, the genera Sphingomonas and Pseudomonas, that are capable of degrading n-alkanes having 4 to 20 carbon atoms.

## Description

### Field of the invention:

The present invention relates to a process for preparing *N*-substituted 4-hydroxypiperidines that are useful as intermediates for the preparation of several pharmaceutical products and agricultural chemicals, wherein an oxygen atom is inserted regioselectively into the corresponding *N*-substituted piperidines by use of biocatalysts.

### Description of the Prior Art:

4-Hydroxypiperidine and *N*-substituted 4-hydroxypiperidines are useful intermediates for the syntheses of several pharmaceuticals, agrochemicals, and the like.

In practice it is often advantageous, if not required, to use 4-hydroxypiperidine in its *N*-protected form.

It is known that *N*-substituted 4-hydroxypiperidines can be prepared by reduction of the corresponding *N*-substituted 4-piperidones [2776293 (01. Jan. 1957); US 2767190 (16. Oct. 1956); GB629196; HU 204035; Okano, T.; Matsuoka, M.; Konishi, H.; Kiji, J. *Chem. Lett.,* **1987,** 181-184; Kostochka, L. M.; Belostotskii, A. M.; Skoldinov, A. P., *J. Org. Chem. USSR (Engl. Transl.)*, **1982,** *18,* 2315-2316; McElvain, S. M.; McMahon, R. E., *J. Amer. Chem. Soc.* **1947,** *71,* 901-906; Bolyard, N. W., *J. Amer. Chem. Soc*. **1930,** *52,* 1030] that were synthesized from ethyl acrylate and alkylamine *via* carboxyethylation, condensation, and decarboxylation [Grob, C. A.; Brenneisen, P., *Helv. Chim. Acta*, **1958,** *41,* 1184; Brookes, P.; Walker, J, *J. Amer. Chem. Soc*. **1957,** *79*, 3173-3175; McElvain, S. M.; McMahon, R. E., *J. Amer. Chem. Soc.* **1949,** *71,* 901-906; Kuettel, G. M.; McElvain, S. M., *J. Amer. Chem*. *Soc.,* **1931,** 2692-2696; Bolyard, N. W., *J. Amer. Chem*. *Soc*. **1930**, *52,* 1030; Dickerman, S. C.; Lindwall, H. G., *J. Org. Chem.,* **1949,** *14,* 530-536]. Such processes involve muti-step syntheses and have the separation problem in each step.

It is also known that 4-hydroxypiperidine and *N*-substituted hydroxypiperidines can be prepared by hydrogenation of 4-hydroxypyridine [Schaefgen, J. R.; Koontz, F. H.; Tietz, R. F. *J. Polymer Sci*., **1959,***40*, 377; Hall, Jr., H. K., *J. Amer. Chem. Soc*., **1958,** *80,* 6412-6420; Fr. 1491127 (04 Aug 1967)] and *N*-substituted 1*H*-pyridin-4-one [Coan etal *J. Amer. Chem. Soc*., **1956,** *78,* 3701], respectively. However, the yields are low.

4-Hydroxypiperidine can be prepared from 3-hydroxy-glutaronitrile *via* hydrogenation and cyclization [Bowden; Green, *J. Chem. Soc.,* **1956,** *78,* 370]. *N*-Benzyl 4-hydroxypiperidine can be prepared from benzyl-but-3-enyl-amine and formaldehyde [McCann, S. F.; Overman, L. E., *J. Amer. Chem*. *Soc*., **1987,** *109,* 6170-6114], and it can also be prepared from *N*-benzylammonium trifluoroacetate, allyl-trimethyl-silane, and formaldehyde [Larsen, S. D.; Grieso, P. A.; Fobare, W. F., *J. Amer*. *Chem*. *Soc.,* **1986,** *108,* 3512-3513]. However, all such preparations are not practical for a large scale.

4-Hydroxypiperidine can be synthesized from *N*-trimethylsilanyl-1,2,5,6-tetrahydropyridine *via* hydroxylation of C=C bond [Dicko, A.; Montury, M.; Baboulene, M., *Tetrahedron Lett*., **1987,** *28,* 6041-6044], however, 3-hydroxypiperidine was obtained as main product. Hydroxylation of C=C bond in *N*-benzyloxylcarbonyl-1,2,5,6-tetrahydropyridine gave *N*-benzyloxylcarbonyl 4-hydroxypiperidine, but 3-hydroxylated compound was also formed [Brown, H. C.; Prasad, J. V. N. V. *J. Amer*. *Chem. Soc*., **1986,** *108,* 2049-2054; Brown, H. C.; Prasad, J. V. N. V.; Zee, S.-H. *J. Org. Chem.,* **1985,** *50,* 1582-1589].

Regioselective hydroxylation of *N*-substituted piperidines could provide a simple process for preparing *N*-substituted 4-hydroxypiperidines. However, this reaction cannot be carried out with classic chemical method.

Enzymatic hydroxylation of piperidines with fungi are known: hydroxylation of *N*-benzoyl piperidine with *Beauveria sulfurescens* ATCC 7159 afforded 6.5% [Archelas, A.; Furstoss, R.; Srairi, D.; Maury, G., *Bull. Soc. Chem. Fr.* **1986,** 234-238], 19% [Johnson, R. A.; Herr, M. E.; Murray, H. C.; Fonken, G. S., *J. Org. Chem.,* **1968,** 3187; GB 1140055 (15 Jan. 1969)], and 20% [Holland, H. L.; Morris, T. A.; Nava, P. J.; Zabic, M. *Tetrahedron*, **1999,** *55,* 7441-7460] of *N*-benzoyl 4-hydroxypiperidine. In another report [SU 1822886 (23.06.93); Parshikov, I. A.; Modyanova, L. V.; Dovgilivich, E. V.; Terent'ev, P. B.; Vorob'eva, L. I.; Grishina, G. V. *Chem. Heterocycl*. *Compd. (Engl. Transl.),* **1992,** 28, 159-162], hydroxylation of *N*-benzoyl piperidine with *Beauveria bassiana* VKM F-3111D resulted a mixture of 4-hydroxy- and 3-hydroxy-piperidine; hydroxylation with *Penicitlium simplicissimum* gave a mixture of 4-hydroxy- and 2-hydroxy-piperidine; hydroxylation with *Cunninghamella verticillata* VKPM F-430, *Aspergillus awamori* VKM F-758, and *Aspergillus niger* VKM F-1119, respectively, afforded 19%, 34%, and 80% of *N*-benzoyl 4-hydroxypiperidine, respectively. Hydroxylation of *N*-benzyloxycarbonyl piperidine was known with *Beauveria sulfurescens* ATCC 7159, affording 33% of *N*-benzyloxycarbonyl 4-hydroxypiperidine [Aitken, S. J.; Grogan, G.; Chow, C. S.-Y.; Turner, N. J.; Flitsch, S. L., *J. Chem*. *Soc. Perkin trans. 1,* **1998,** 3365-3370; Flitsch, S. L.; Aitken, S. J.; Chow, C. S.-Y.; Grogan, G.; Staines, A., *Bioorg. Chem.* **1999,** *27,* 81-90]. Hydroxylation of *N*-arylpiperidines with *Beauveria sulfurescens* ATCC 7159 gave 20-66% of the corresponding *N*-aryl-4-hydroxypiperidines [Floyd, N.; Munyemana, F.; Roberts, S. M.; Willetts, A. J., *J. Chem. Soc*. *Perkin trans. 1*, **1993,** 881]. However, besides the problems of low yields and formation of by-products, all such processes with fungi as biocatalysts are not practical, since the concentration of product is too low (< 0.1 g/L), the reaction time is too long (3-6 days), and separation of the product from biotransformation mixture with fungi is very difficult.

### Summary of the invention

This invention provides a process for a practical preparation of *N*-substituted 4-hydroxypiperidine, wherein an oxygen atom is inserted regioselectively into the corresponding *N*-substituted piperidine, by use of a bacterium degrading alkanes or alicyclic hydrocarbons, or a prokaryotic host-organism having the gene(s) necessary for the hydroxylation derived from the said bacterium, or an enzyme having hydroxylation activity derived therefrom.

More specifically, the bacterium used is selected from the group consisting of strains degrading n-alkanes or mono-alicycles. Prefered are *n*-alkane-degrading strains, such as the isolates *Sphingomonas sp.* HXN-200, HXN-100, HXN-1400, HXN-1500, PN3, PN21, PN26, PN27, PN32, S69, S70, *Pseudomonas putid*α P1, and *Pseudomonas oleovorans* GPo1 (ATCC 29347), and mono-alicycles-degrading strains, such as cyclohexane-degrading strain LD-5. The invention includes the use of the recombinant bacteria having the gene(s) necessary for the hydroxylation derived from the strain degrading alkanes or alicyclic hydrocarbons. Preferred are the recombinant *Escherichia coli* strains, such as *Escherichia coli* GEc137 (pGEc47).

The biotransformation is performed *in vivo* with resting cells as biocatalysts, *in vivo* with growing cells as biocatalysts, or *in vitro* with crude cell extracts or enzyme preparations that are purified or partially purified as biocatalysts.

The biocatalysts can be immobilized on or in a water-insoluble carrier or support system.

The biotransformation is performed in aqueous medium or in mutiphase media possibly containing two or more of the following: a solid phase, an aqueous phase, an organic phase, or a gasiform phase.

The reaction temperature is 5-50°C, preferably at 20-40° and the pH of the medium is 4-10, preferably 6-8.

The isolation of *N*-substituted 4-hydroxypiperidine is performed by means of extraction, by separation techniques such as chromatography with an inorganic, organic, or synthetic adsorbent used as a support, or by membrane filtration.

In a preferred embodiment, *N*-benzyl-, *N*-benzyloxycarbonyl-, *N*-phenoxycarbonyl-, *N-tert*-butoxycarbonyl-, and *N*-benzoyl- 4-hydroxypiperidine were prepared by regioselective insertion of an oxygen atom into *N*-benzyl-, *N* benzyloxycarbonyl-, *N*-phenoxycarbonyl-, *N-tert*-butoxycarbonyl-, and *N*-benzoyl-piperidine, repectively, by use of *Sphingomonas sp.* HXN-200, or HXN-100, or HXN-1400, or HXN-1500, or PN3, or PN21, or PN26, or PN27, or PN32, or S69, or S70, or *Pseudomonas putid*α P1, or *Pseudomonas oleovorans* GPo1 (ATCC 29347), or cyclohexane-degrading strain LD-5, or other bacteria degrading n-alkanes or mono-alicycles containing 4 or more C atoms, or a prokaryotic host-organism having the gene(s) necessary for the hydroxylation derived from the said bacterium, or an enzyme having hydroxylation activity derived therefrom.

*N*-substituted 4-hydroxypiperidine obtained by this process can be easily converted into 4-hydroxypiperidine by deprotection.

Thus, the invention here provides a useful method for the preparation of *N*-substituted 4-hydroxypiperidines and 4-hydroxypiperidine.

### Description of the invention

Here we have developed a process for a practical preparation of *N*-substituted 4-hydroxypiperidine, wherein an oxygen atom is inserted regioselectively into the corresponding *N*-substituted piperidine, by use of a bacterium degrading alkanes or alicyclic hydrocarbons, or a prokaryotic host-organism having the gene(s) necessary for the hydroxylation derived from the said bacterium, or an enzyme having hydroxylation activity derived therefrom.

For finding appropriate biocatalysts for this reaction we have screened many bacteria by use of a miniaturised screening system on a microtiter plate. In a screening procedure demonstrated in example 1, 96 of alkane-degrading strains were grown on vapour of a mixture of *n*-hexane/*n*-octane/*n*-decane*/n*-dodecane/*n*-tetradecane (1:1:1:1:1) in agar-based mineral growth medium on a microtiter plate for 3-6 days. The cells were harvested and resuspended in 150 ?l of 50 mM phosphate buffer (pH = 7.2) containing 1-2% of glucose on a microtiter plate. *N*-Substituted piperidine (0.1-1.0 mM) was added, and the mixture was shaken at 200 rpm and at 25°C for 2 h. The formation of *N*-substituted 4-hydroxypiperidine was determined by high performance liquid chromatography (HPLC) coupled with MS detection.

It has been found that many alkane-degrading bacteria are able to catalyse regioselectively the hydoxylation of *N*-substituted piperidine to give the corresponding 4-hydroxypiperidine. Examples of these bacteria are, as shown in table 1, the isolates *Sphingomonas sp.* HXN-200, HXN-100, HXN-1400, HXN-1500, PN3, PN21, PN26, PN27, PN32, S69, S70, *Pseudomonas putida* P1, and *Pseudomonas oleovorans* GPo1 (ATCC 29347).

It has been found that many strains degrading alicyclic hydrocarbons are able to catalyse regioselectively the hydoxylation of *N*-substituted piperidine to give the corresponding 4-hydroxypiperidine. One example of these bacteria is cyclohexane-degrading strain LD-5.

It has also been found that the biocatalysts can be a prokaryotic host-organism having the gene(s) necessary for the hydroxylation from the strain degrading alkanes or alicyclic hydrocarbons. The recombinant *Escherichia coli* GEc137 (pGEc47), for example, is a suitable catalyst for hydroxylation of *N*-substituted piperidine affording *N*-substituted 4-hydroxypiperidine.

It has been found that hydoxylation of *N*-substituted piperidines can be catalysed by an enzyme having hydroxylation activity derived from the said bacteria to give the corresponding 4-hydroxypiperidine.

The biotransformation can be performed *in vivo* with resting cells as biocatalysts, *in vivo* with growing cells as biocatalysts, or *in vitro* with purified enzymes or crude cell extracts as biocatalysts.

The biocatalysts can be immobilized on or in a water-insoluble carrier or support system.

The biotransformation can be carried out in aqueous medium. It can also be performed in mutiphase media possibly containing two or more of the following: a solid phase, an aqueous phase, an organic phase, or a gasiform phase. Organic solvents with high LogP values can be used as organic phase. This includes alkanes with 5 or more C atoms, dialkyl ethers with 4 or more C atoms, carboxylic esters with 4 or more C atoms, and aromatic hydrocarbons. An example of a suitable organic solvent is hexadecane.

The enzymatic hydroxylations can be carried out, although this is no critical parameter, at a temperature of 5-50°C, preferably at 20-40°C. The. pressure can vary within wide limits. In practice the biotransformation is performed at atmospheric pressure. The pH of the reaction medium can be between 4 and 10, preferably between 6 and 8.

The product can be separated by chromatographic techniques with an inorganic, organic, or synthetic adsorbent used as a support. The suitable adsorbents are, for instance, aluminium oxide and silica gel. The product can be also isolated by membrane filtration.

The product can be also separated by means of extraction, wherein the substrate is first recovered from the reaction mixture by extraction with less polar solvent, the remaining reaction mixture is adjusted to pH =10-12, and the product is extracted out with more polar solvent. The suitable extraction agent used is selected from the group consisting of alkanes with 5 or more C atoms, dialkyl ethers with 4 or more C atoms, chlorine-containing alkanes with 3 or fewer C atoms, alkyl aromatics with 7-10 C atoms, and carboxylic esters with 3 or more C atoms. Examples of particularly suitable extraction agents are hexane and ethyl acetate, as apolar and polar solvent, respectively.

It has been found that *N*-substituted 4-hydroxypiperidine can be prepared by regioselective insertion of an oxygen atom into the corresponding *N*-substituted piperidine by use of *Sphingomonas* HXN-200 (isolated by Plaggemeier, Th.; Schmid, A.; Engesser, K. at University of Stuttgart; in the strain collection of Institute of Biotechnology, ETH Zurich). The cells of *Sphingomonas sp.* HXN-200 was prepared in large scale by growing in E2 medium either with n-octane as carbon source or with glucose as carbon source followed by induction of the alkane oxidation system with dicyclopropyl ketone (DCPK) or n-octane. The cells can be stored at -80°C for several months and used as normal chemical reagent in a bioconversion with resting cells.

In bioconversion with resting cells of HXN-200, *N*-substituted piperidine (2-10 mM) was added to 10 ml of cell suspension (4.0 g/L) in 50 mM K-phosphate buffer (pH 8.0) containing glucose (0 or 2%), and the mixture was shaken at 30°C for 5 h. The reaction was followed by analytical HPLC: samples were taken out directly from the reaction mixture at different times, the cells were removed by centrifugation, and the supernatants were analysed by analytical HPLC.

HPLC analytical methods were established by use of a Hypersil BDS-C18 (5 µm, 125 mm x 4 mm) column, a mixture of acetonitrile/10 mM K-phosphate buffer (pH 7.0) as eluent, flow at 1.0 ml/min., and detections at 210, 225, and 254 nm. Retention time of *N*-benzyl 4-hydroxypiperidine: 3.0 min.; retention time of *N*-benzyl piperidine: 5.2 min. [acetonitrile/10 mM K-phosphate buffer (pH 7.0) 15:85]; Retention time of *N*-benzyloxycarbonyl 4-hydroxypiperidine: 1.9 min.; retention time of *N*-benzyloxycarbonyl piperidine: 8.5 min. [acetonitrile/10 mM K-phosphate buffer (pH 7.0) 45:55]; Retention time of *N*-phenoxycarbonyl 4-hydroxypiperidine: 1.7 min.; retention time of *N*-phenoxycarbonyl piperidine: 6.6 min. [acetonitrile/10 mM K-phosphate buffer (pH 7.0) 45:55]; Retention time of *N-tert*-butoxycarbonyl 4-hydroxypiperidine: 1.5 min.; retention time of *N-tert*-butoxycarbonyl piperidine: 5.6 min. [acetonitrile/10 mM K-phosphate buffer (pH 7.0) 45:55]; Retention time of *N*-benzoyl 4-hydroxypiperidine: 1.4 min.; retention time of *N*-benzoyl piperidine: 4.9 min. [acetonitrile/10 mM K-phosphate buffer (pH 7.0) 30:70].

A procedure for standard work-up was established: the cells were removed by centrifugation, the supernatants were adjusted to pH = 10-12 by the addition of KOH followed by extraction with ethyl acetate. The organic phase was dried over MgSO₄, filtered, and the solvent evaporated.

The products were purified by column chromatography either on aluminium oxide or silica gel. Their structures were identified by ¹H- and ¹³C-NMR and MS spectra.

Hydroxylation of *N*-benzyl piperidine with resting cells (4g/L) of HXN-200 gave high activity. As shown in table 2, the average activity in the first 30 min. reaches 19-20 U/g CDW for hydroxylation of 5-10 mM of *N*-benzyl piperidine. It has been found that addition of 2% glucose in the reaction mixture increases the yield. Hydroxylation of *N*-benzyl piperidine (5 mM) with resting cells in the presence of 2% of glucose resulted 100 % *N*-benzyl 4-hydroxypiperidine in 5 h.

It has been found that hydroxylation of *N*-benzyloxycarbonyl piperidine (4-5 mM) with resting cells (4g/L) of HXN-200 gave an activity of 12-14 U/g CDW. As shown in table 3, addition of 2% glucose increased the yield at 5 h of *N*-benzyloxycarbonyl 4-hydroxypiperidine from 24% to 57% and from 34% to 57% for hydroxylation of 4 mM and 5 mM of *N*-benzyloxycarbonyl piperidine, respectively (table 3).

As shown in table 4, hydroxylation of *N*-phenoxycarbonyl piperidine (5-8 mM) with resting cells (4g/L) of HXN-200 gave an activity of 18-20 U/g CDW. 95% of *N*-phenoxycarbonyl 4-hydroxypiperidine was formed by hydroxylation of 8 mM of *N*-phenoxycarbonyl piperidine in the presence of 2% glucose at 5 h.

It has also been found that hydroxylation of *N-tert*-butoxycarbonyl piperidine (5-8 mM) with resting cells (4g/L) of HXN-200 in the presence of 2% glucose gave 51-94% of *N-tert*-butoxycarbonyl 4-hydroxypiperidine at 5 h. The activity is very high: 21-30 U/g CDW.

As shown in table 6, hydroxylation of *N*-benzoyl piperidine (2-4 mM) with resting cells (4g/L) of HXN-200 in the presence of 2% glucose for 5 h afforded 57-99% of *N*-benzoyl 4-hydroxypiperidine with an activity of 2.7-4.8 U/g CDW.

It has been found that *Escherichia coli* GEc137 (pGEc47) [described by Eggink, G. et al, in *J. Biol*. *Chem.* **1987,** *262,* 17712; in strain collection of Institute of Biotechnology, ETH Zurich], a recombinant strain carrying the genes for a multicomponent alkane hydroxylase from *Pseudomonas oleovorans* GPo1, catalyses the hydroxylation of *N*-benzyl piperidine to *N*-benzyl 4-hydroxypiperidine. In example 3, *Escherichia coli* GEc137(pGEc47) was grown on glucose in M9 medium followed by induction with DCPK. Cells were harvested and resuspended to 2.5 g/L in 50 mM K-phosphate buffer (pH 7.2) containing glucose (2% w/v). Biocoversion of *N*-benzyl piperidine (2 mM) with these cells at 30°C for 5 h gave 80% of *N*-benzyl-3-4-hydroxypiperidine.

It has been found that *N*-substituted 4-hydroxypiperidines can be easily prepared by biohydroxylation of the corresponding *N*-substituted piperidines in a shaking flask. Example 4 demonstrated the hydroxylation of *N*-benzyloxycarbonyl piperidine (43.8 mg, 0.20 mmol) with resting cells (4.0 g/L) of *Sphingomonas sp.* HXN-200 in 100 ml of 50 mM K-phosphate buffer (pH 8.0) containing glucose (2%) in a 500 ml shaking flask. Biotransformation at 200 rpm and 30°C for 3 h formed 96% of *N*-benzyloxycarbonyl 4-hydroxypiperidine. Standard work-up and column chromatography on silica gel (R_{f} = 0.12, *n*-hexane/ethyl acetate 1:1) afforded 70.2% (33.0 mg) of *N*-benzyloxycarbonyl 4-hydroxypiperidine.

Similarly, as demonstrated in example 5, bioconversion of *N*-phenoxycarbonyl piperidine (143.5 mg, 0.70 mmol) in 100 ml of cell suspension (4.0 g/L) of HXN-200 in 50 mM K-phosphate buffer (pH 8.0) containing glucose (2%) gave 91% conversion to *N*-phenoxycarbonyl 4-hydroxypiperidine after shaking at 200 rpm and 30°C for 4 h. Standard work-up and column chromatography on silica gel (R_{f} = 0.11, *n*-hexane/ethyl acetate 1:1) afforded 83.2% (143.6 mg) of *N*-phenoxycarbonyl 4-hydroxypiperidine.

In example 6, hydroxylation of *N-tert*-butoxycarbonyl piperidine (92.5 mg, 0.50 mmol) was performed with resting cells (4.0 g/L) of HXN-200 in 100 ml of 50 mM K-phosphate buffer (pH 8.0) containing glucose (2%). Shaking at 200 rpm and at 30°C for 2 h formed 96% of *N-tert-*butoxycarbonyl 4-hydroxypiperidine. 69.5% (69.3 mg) of pure product was obtained after standard work-up and column chromatography on silica gel (R_{f} = 0.20, *n*-hexane/ethyl acetate 1:1).

Similarly, bioconversion of *N*-benzoyl piperidine was carried out in 100 ml of cell suspension (4.0 g/L) of HXN-200 in 50 mM K-phosphate buffer (pH 8.0) containing glucose (2%) in a 500 ml shaking flask at 200 rpm and 30°C for 5 h. 83% and 62% conversion to *N*-benzoyl 4-hydroxypiperidine were achieved in hydroxylation of 37.8 mg (0.20 mmol) and 56.7 mg (0.30 mmol) of *N*-benzoylpiperidine, respectively. Standard work-up and column chromatography on silica gel (R_{f} = 0.14, ethyl acetate) gave 71.5% (29.3 mg) and 52.2% (32.1 mg) of *N*-benzoyl 4-hydroxypiperidine, respectively, as demonstrated in example 7 and 8.

It has been found that bioconversion of *N*-substituted piperidines to the corresponding *N*-substituted 4-hydroxypiperidines can be easily performed in a bioreactor. As shown in example 9, preparation of *N*-benzyl 4-hydroxypiperidine was carried out by hydroxylation of *N*-benzyl piperidine with resting cells (4.0 g/L) of *Sphingomonas sp.* HXN-200 in 2 L scale. Hydroxylation of *N*-benzyl piperidine (1.75 g, 10 mmol) for 4 h formed nearly 100% of *N*-benzyl 4-hydroxypiperidine. Standard work-up and column chromatography on silica gel (R_{f} = 0.20, ethyl acetate/MeOH 8:2) gave 83% (1.50 g) of pure product as white powder. White crystals were obtained by crystallization from ethyl acetate/hexane (1/3).

It has been found that product concentration can be easily increased by use of cell suspension with higher density. In Example 10, preparation of *N*-benzyl-4-hydroxypiperidine was performed by hydroxylation of *N*-benzyl piperidine with cell suspension of HXN-200 with density of 10.2 g/L in 1 L scale in a bioreactor. Bioconversion of *N*-benzyl piperidine (2.63 g, 15 mmol) for 5.2 h formed 98% of *N*-benzyl 4-hydroxypiperidine. Standard work-up and column chromatography gave 2.07 g (72%) of pure *N*-benzyl 4-hydroxypiperidine as white powder.

It has been found that hydroxylation of *N*-substituted piperidines can be easily carried out with growing cells as biocatalyst. Example 11 demonstrated hydroxylation of *N*-benzyl piperidine with growing cells of *Sphingomonas sp.* HXN-200 in 1 L scale. The cells were grown in E2 medium first on glucose and then on n-octane to a cell density of 6.2 g/L. *N*-benzyl piperidine (0.875 g, 5 mmol) was added, the mixture was stirred at 1536 rpm at 30°C with air introduction at 2 L/min. During bioconversion, *n*-octane vapour was still introduced and the cells were still grown. Additional substrate was added at 30 min. (0.875 g, 5 mmol), 60 min. (0.875 g, 5 mmol), and 90 min. (0.875 g, 5 mmol). 85% of *N*-benzyl 4-hydroxypiperidine were formed at 2 h, and 2.866 g (75%) of pure product were yielded after standard work-up and column chromatography.

It has been found that hydroxylation of *N*-substituted piperidines can be carried out with cell-free extracts as biocatalyst. Hydroxylation of *N*-benzyl piperidine with cell-free extracts of *Sphingomonas sp.* HXN-200 was demonstrated in example 12. Cells of HXN-200 were suspended in 10 ml of Tris-HCl buffer (pH = 7.5) to a density of 20 g/L. After passage through the French press three times, the cell debris was removed by centrifugation at 45000 rpm for 45 min. giving soluble cell-free extracts containing no membrane proteins. To this cell-free extracts was added NADH (5 mM) and *N*-benzyl piperidine (5 mM). The mixture was shaken at 200 rpm and at 30°C for 1 h afforded 90% of *N*-benzyl-4-hydroxypiperidine. This also demonstrates that the enzyme in HXN-200 catalysing this reaction is not membrane-bound.

It has been found that hydroxylation of *N*-substituted piperidine to *N*-substituted 4-hydroxypiperidine can be catalysed by strains degrading alicyclic hydrocarbons. Example 13 demonstrated hydroxylation of *N*-benzyl piperidine with cyclohexane-degrading bacterium LD-5 (isolated by Li, Z. and Deutz, W., ETH Zurich; in the strain collection of Institute of Biotechnology, ETH Zurich). The strain was grown on vapour of cyclohexane diluted 10 times by air in 1/4 of Evans medium. The cells were harvested and resuspened to 5 g/L in 50 mM K-phosphate buffer (pH 7.2) containing glucose (2%w/v). Bioconversion of *N*-benzyl piperidine(5 mM) at 30°C for 2 h afforded 60% of *N*-benzyl-4-hydroxypiperidine.

The specific examples given herein are intended merely as an illustration of the invention and should not be construed as a restriction of the scope of the invention.

### Examples

### Example 1: Screening of biocatalyst for hydroxylation of N-benzyl piperidine to N-benzyl 4-hydroxypiperidine on microscale

Alkane-degrading strains were grown on vapour of a mixture of *n*-hexane/*n*-octane/*n*-decane/*n*-dodecane/*n*-tetradecane (1:1:1:1:1) in agar-based Evans medium on a microtiter plate for 3-6 days. The cells were resuspended in 150 µl of 50 mM phosphate buffer (pH = 7.2) containing 100 mM glucose and 150 µM *N*-benzyl piperidine on a microtiter plate. The mixture was shaken at 200 rpm and at 25°C for 2 h. Cells were removed by centrifugation, and the supernatants were analysed for the formation of *N*-benzyl 4-hydroxypiperidine by HPLC-MS.

Conditions for HPLC-MS analysis: Nucleosil 100-5 C18 pre-column; acetonitrile/10 mM K-phosphate buffer (pH 7.0) 1/9 for 2 min., then gradient to 46/54 till 5 min.; flow at 1.0 ml/min.; MS detection at 176 and 192; retention time of *N*-benzyl 4-hydroxypiperidine: 1.4 min.; retention time of *N*-benzyl piperidine: 3.6 min. The results were summarized in table 1.

**Table 1:**

| Hydroxylation of *N*-benzyl piperidine to *N*-benzyl 4-hydroxypiperidine with several alkane-degrading bacteria | | |
|---|---|---|
| Entry | Strains¹ | Relative activity² |
| 1 | *Sphingomonas sp*. HXN-200 | 1 |
| 2 | HXN-100 | 2.1 |
| 3 | HXN-1400 | 0.7 |
| 4 | HXN-1500 | 1.3 |
| 5 | PN 3 | 0.5 |
| 6 | PN 21 | 0.4 |
| 7 | PN 26 | 0.2 |
| 8 | PN 27 | 0.2 |
| 9 | PN 32 | 0.3 |
| 10 | S 69 | 0.2 |
| 11 | S 70 | 0.4 |
| 12 | *Pseudomonas putida* P1 | 0.2 |
| 13 | *Pseudomonas oleovorans* GPo1 (ATCC | 0.2 |

| | | |
|---|---|---|
| ¹ Strains 1-12 are isolates by use of *n*-hexane or *n*-octane as carbon source; all strains are in the strain collection of Institute of Biotechnology, ETH Zurich. | | |
| ²Activity was compared with that of *Sphingomonas sp.* HXN-200. | | |

### Example 2: Hydroxylation of N-benzyl-, N-benzyloxycarbonyl-, N-phenoxycarbonyl, N-tert-butoxycarbonyl, and N-benzoyl- piperidine with resting cells of Sphingomonas sp. HXN-200

*Sphingomonas sp.* HXN-200 (isolated by Plaggemeier, Th.; Schmid, A.; Engesser, K. at University of Stuttgart; in the strain collection of Institute of Biotechnology, ETH Zurich) was inoculated in 2 L of E2 medium with vapour of *n*-octane as carbon source and grown at 30°C, the cells were harvested at a cell density of 2-10 g/L and stored at -80°C.

In a general procedure, *N*-substituted piperidine (2-10 mM) was added to 10 ml cell suspension (4.0 g/L) of HXN-200 in 50 mM K-phosphate buffer (pH 8.0) containing glucose (0 or 2%), and the mixture was shaken at 30°C for 5 h. The reaction was followed by analytical HPLC: samples were taken out directly from the reaction mixture at different times, the cells were removed by centrifugation, and the supernatants were analysed by analytical HPLC.

HPLC analyses were performed on a Hypersil BDS-C18 (5 µm, 125 mm x 4 mm) column with a mixture of acetonitrile/10 mM K-phosphate buffer (pH 7.0) as eluent, flow at 1.0 ml/min., and DAD detections at 210, 225, and 254 nm.

Retention time of *N*-benzyl 4-hydroxypiperidine: 3.0 min.; retention time of *N*-benzyl piperidine: 5.2 min. [acetonitrile/10 mM K-phosphate buffer (pH 7.0) 15:85].

Retention time of *N*-benzyloxycarbonyl 4-hydroxypiperidine: 1.9 min.; retention time of *N*-benzyloxycarbonyl piperidine: 8.5 min. [acetonitrile/10 mM K-phosphate buffer (pH 7.0) 45:55].

Retention time of *N*-phenoxycarbonyl 4-hydroxypiperidine: 1.7 min.; retention time of *N*-phenoxycarbonyl piperidine: 6.6 min. [acetonitrile/10 mM K-phosphate buffer (pH 7.0) 45:55].

Retention time of *N-tert*-butoxycarbonyl 4-hydroxypiperidine: 1.5 min.; retention time of *N-tert*-butoxycarbonyl piperidine: 5.6 min. [acetonitrile/10 mM K-phosphate buffer (pH 7.0) 45:55].

Retention time of *N*-benzoyl 4-hydroxypiperidine: 1.4 min.; retention time of *N*-benzoyl piperidine: 4.9 min. [acetonitrile/10 mM K-phosphate buffer (pH 7.0) 30:70].

The crude product was obtained by standard work-up: the cells were removed by centrifugation, the supernatants were adjusted to pH = 10-12 by the addition of KOH followed by extraction with ethyl acetate. The organic phase was dried over MgSO₄, filtered, and the solvent evaporated.

The products were purified by column chromatography either on aluminium oxide or silica gel, and their structures were identified by 1H- and ¹³C- NMR and MS spectra.
The results are listed in table 2-6.

**Table 2:**

| Hydroxylation of *N*-benzyl piperidine to *N*-benzyl-4-hydroxypiperidine with resting cells (4.0 g/L) of HXN-200 | | | | | | | | |
|---|---|---|---|---|---|---|---|---|
| Entry | Substrate (mM) | Glucose (%) | Activity¹ (U/gCDW) | Prod. (%) | | | | |
| | | | | 0.5 h | 1 h | 2h | 3h | 5h |
| 1 | 2 | | 9.0 | 54 | 68 | 84 | 87 | 89 |
| 2 | 2 | 2 | 12 | 73 | 98 | 100 | | |
| 3 | 5 | | 10 | 25 | 35 | 39 | 40 | 42 |
| 4 | 5 | 2 | 20 | 49 | 77 | 94 | 98 | 100 |

| | | | | | | | | |
|---|---|---|---|---|---|---|---|---|
| ¹ Activity was determined over the first 30 min. | | | | | | | | |

**Table 3:**

| Hydroxylation of *N*-benzoxycarbonyl piperidine to *N*-benzoxycarbonyl 4-hydroxypiperidine with resting cells (4.0 g/L) of HXN-200 | | | | | | | | |
|---|---|---|---|---|---|---|---|---|
| Entry | Substrate (mM) | Glucose (%) | Activity¹ (U/g CDW) | Product (%) | | | | |
| | | | | 0.5 h | 1 h | 2 h | 3 h | 5 h |
| 1 | 2 | 0 | 9.2 | 54 | 70 | 76 | 75 | 81 |
| 2 | 2 | 2 | 12 | 71 | 85 | 90 | 90 | 93 |
| 3 | 3 | 0 | 6.4 | 25 | 41 | 55 | 51 | 52 |
| 4 | 3 | 2 | 13 | 49 | 61 | 65 | 68 | 69 |
| 5 | 4 | 0 | 6.5 | 19 | 26 | 38 | 40 | 42 |
| 6 | 4 | 2 | 14 | 41 | 45 | 55 | 54 | 57 |
| 7 | 5 | 0 | 6.0 | 14 | 20 | 28 | 26 | 34 |
| 8 | 5 | 2 | 12 | 28 | 35 | 47 | 48 | 57 |

| | | | | | | | | |
|---|---|---|---|---|---|---|---|---|
| ¹ Activity was determined over the first 30 min. | | | | | | | | |

**Table 4:**

| Hydroxylation of *N*-phenoxycarbonyl piperidine to *N*-phenoxycarbonyl 4-hydroxypiperidine with resting cells (4.0 g/L) of HXN-200 | | | | | | | | |
|---|---|---|---|---|---|---|---|---|
| Entry | Substrate | Glucose | Activity¹ | Product (%) | | | | |
| | (mM) | (%) | (U/g CDW) | 0.5 h | 1 h | 2 h | 3 h | 5 h |
| 1 | 2 | 0 | 1.7 | 10 | 21 | 35 | 45 | 53 |
| 2 | 2 | 2 | 16 | 93 | >98 | >98 | >98 | >98 |
| 3 | 5 | 0 | 3.8 | 9 | 29 | 33 | 33 | 38 |
| 4 | 5 | 2 | 20 | 46 | 84 | >98 | >98 | >98 |
| 5 | 6 | 0 | 4.6 | 9 | 16 | 26 | 28 | 39 |
| 6 | 6 | 2 | 19 | 37 | 60 | 92 | 93 | >98 |
| 7 | 7 | 0 | 4.8 | 8 | 12 | 19 | 22 | 33 |
| 8 | 7 | 2 | 19 | 31 | 53 | 84 | 91 | >98 |
| 9 | 8 | 0 | 4.8 | 7 | 9 | 16 | 18 | 27 |
| 10 | 8 | 2 | 18 | 26 | 51 | 76 | 88 | 95 |

| | | | | | | | | |
|---|---|---|---|---|---|---|---|---|
| ¹ Activity was determined over the first 30 min. | | | | | | | | |

**Table 5:**

| Hydroxylation of *N-tert*-butoxycarbonyl piperidine to *N-tert-*butoxycarbonyl-4-hydroxypiperidine with resting cells (4.0 g/L) of HXN-200 | | | | | | | | |
|---|---|---|---|---|---|---|---|---|
| Entry | Substrate | Glucose | Activity¹ | Product (%) | | | | |
| | (mM) | (%) | (U/g CDW) | 0.5 h | 1 h | 2 h | 3 h | 5 h |
| 1 | 2 | 0 | 7.7 | 45 | 63 | 86 | 80 | 78 |
| 2 | 2 | 2 | 15 | 86 | 86 | 94 | 94 | 89 |
| 3 | 5 | 0 | 26 | 61 | 76 | 89 | 92 | 93 |
| 4 | 5 | 2 | 29 | 68 | 85 | 94 | 94 | 94 |
| 5 | 6 | 0 | 24 | 47 | 59 | 65 | 72 | 68 |
| 6 | 6 | 2 | 30 | 59 | 85 | 92 | 93 | 94 |
| 7 | 7 | 0 | 20 | 34 | 48 | 50 | 52 | 54 |
| 8 | 7 | 2 | 21 | 36 | 45 | 48 | 53 | 56 |
| 9 | 8 | 0 | 7 | 10 | 13 | 19 | 15 | 18 |
| 10 | 8 | 2 | 23 | 33 | 44 | 52 | 49 | 51 |

| | | | | | | | | |
|---|---|---|---|---|---|---|---|---|
| ¹Activity was determined over the first 30 min. | | | | | | | | |

**Table 6:**

| Hydroxylation of *N*-benzoyl piperidine to *N*-benzoyl 4-hydroxypiperidine with resting cells (4.0 g/L) of HXN-200 | | | | | | | | |
|---|---|---|---|---|---|---|---|---|
| Entry | Substrate (mM) | Glucose (%) | Activity¹ (U/gCDW) | Prod. (%) | | | | |
| | | | | 0.5 | 1 h | 2 h | 3 h | 5 h |
| 1 | 2 | | 4.0 | 24 | 39 | 53 | 55 | 57 |
| 2 | 2 | 2 | 4.3 | 26 | 57 | 89 | 97 | 99 |
| 3 | 3 | 0 | 2.8 | 11 | 16 | 22 | 25 | 29 |
| 4 | 3 | 2 | 4.3 | 17 | 32 | 57 | 74 | 90 |
| 5 | 4 | 0 | 1.7 | 5 | 8 | 11 | 13 | 15 |
| 6 | 4 | 2 | 2.7 | 8 | 15 | 28 | 41 | 57 |

| | | | | | | | | |
|---|---|---|---|---|---|---|---|---|
| ¹ Activity was determined over the first 30 min. | | | | | | | | |

### Example 3: Preparation of N-benzyl 4-hydroxypiperidine by hydroxylation of N-benzyl piperidine with resting cells of Escherichia coli GEc137 (pGEc47)

*Escherichia coli* GEc137(pGEc47) (described by Eggink, G. et al, in *J. Biol. Chem.* **1987,** *262,* 17712; in strain collection of Institute of Biotechnology, ETH Zurich) was inoculated in M9 medium with glucose as carbon source and grown at 37°C for 10 h to a cell density of 0.2 g/L. Induction was then made by adding DCPK to a concentration of 2 mM. Cells were harvested at a cell density of 0.3 g/L, and resuspended to 2.5 g/L in 50 mM K-phosphate buffer (pH 7.2) containing glucose (2% w/v). *N*-Benzylpiperidine (2 mM) was added and the mixture was shaken at 30°C for 5 h. Analytical and isolation procedures were as described above. 80% of *N*-benzyl-4-hydroxypiperidine was obtained.

### Example 4: Preparation of N-benzyloxycarbonyl 4-hydroxypiperidine by hydroxylation of N-benzyloxycarbonyl piperidine with resting cells of Sphingomonas sp. HXN-200

*N*-Benzyloxycarbonyl piperidine (43.8 mg, 0.20 mmol) was added to 100 ml of cell suspension (4.0 g/L) of *Sphingomonas sp.* HXN-200 in 50 mM K-phosphate buffer (pH 8.0) containing glucose (2%) in a 500 ml shaking flask. The mixture was shaken at 200 rpm and 30°C and the bioconversion was followed by analytical HPLC. The reaction was stopped at 3 h with 96% conversion to *N*-benzyloxycarbonyl 4-hydroxypiperidine. Standard work-up. and column chromatography on silica gel (R_{f} = 0.12, *n*-hexane/ethyl acetate 1:1) afforded 33.0 mg (70.2%) of *N*-benzyloxycarbonyl 4-hydroxypiperidine.
¹H-NMR (CDCl₃): δ 7.37-7.12 (*m*, 5 H, aromatic H), 5.12 (s, 2 H, PhCH₂), 3.97-3.79 (m, 3 H, H_{A}-C(2), H_{A}-C(6), and ), H-C(4)), 3.21-3.08 *(ddd,* 2 H, *J* = 13.6, 9.4, and 3.5 Hz, H_{B}-C(2), H_{B}-C(6)), 1.91-1.82 (*m*, 2 H, H_{A}-C(3), H_{A}-C(5)), 1.57-1.39 (*ddt,* 2 H, *J* = 13.0, 9.0, and 4.1 Hz, H_{B}-C(3), H_{B}-C(5)), 1.65 ppm(*s*, 1 H, OH).
¹³CNMR (CDCl₃): δ 155.28 (*s*, CO); 136.80 (*s*), 128.50 (*d*), 128.00 (*d*), 127.86 (*d*) (aromatic C); 67.41 (*d*, C-4); 67.13 (*t*, OCH₂Ph); 41.35 (*t*, C-2, C-6); 34.06 ppm (*t*, C-3, C-5).
MS (80eV): m/e 236 (100%, M+1), 222 (17%), 192 (76%), 144 (8%), 102 (17%).

### Example 5: Preparation of N-phenoxycarbonyl 4-hydroxypiperidine by hydroxylation of N-phenoxycarbonyl piperidine with resting cells of Sphingomonas sp. HXN-200

*N*-Phenoxycarbonyl piperidine (143.5 mg, 0.70 mmol) was added to 100 ml of cell suspension (4.0 g/L) of *Sphingomonas sp.* HXN-200 in 50 mM K-phosphate buffer (pH 8.0) containing glucose (2%) in a 500 ml shaking flask. The mixture was shaken at 200 rpm and 30°C and the bioconversion was followed by analytical HPLC. The reaction was stopped at 4 h with 91% conversion to *N*-phenoxycarbonyl 4-hydroxypiperidine. Standard work-up and column chromatography on silica gel (R_{f} = 0.11, *n*-hexane/ethyl acetate 1:1) gave 143.6 mg (83.2%) of *N*-phenoxycarbonyl 4-hydroxypiperidine.
¹H-NMR (CDCl₃): δ 7.40-7.06 (*m*, 5 H, aromatic H); 4.10-3.85 (*m*, 3 H, H_{A}-C(2), H_{A}-C(6), H-C(4)), 3.28 (*s*, *br.,* 2 H, H_{B}-C(2), H_{B}-C(6)), 1.98-1.85 (*m*, 2 H, H_{A}-C(3), H_{A}-C(5)), 1.66-1.48 *(ddt,* 2 H, *J* = 13.0, 8.8, and 4.1 Hz, H_{B}-C(3), H_{B}-C(5)), 1.81 ppm (*s*, 1 H, OH).
¹³C-NMR (CDCl₃): δ 153.75 (*s*, CO); 151.42 (*s*), 129.26 (*d*), 125.25 (*d*), 121.73 (*d*) (aromatic C); 67.08 *(d,* C-4); 41.62 (*t*, C-2, C-6); 33.97 ppm (*t*, C-3, C-5).
MS (80eV): m/e 222 (100%, M+1), 206 (24%).

### Example 6: Preparation of N-tert-butoxycarbonyl 4-hydroxypiperidine by hydroxylation of N-tert-butoxycarbonyl piperidine with resting cells of Sphingomonas sp. HXN-200

*N-tert*-Butoxycarbonyl piperidine (92.5 mg, 0.50 mmol) was added to 100 ml of cell suspension (4.0 g/L) of *Sphingomonas sp.* HXN-200 in 50 mM K-phosphate buffer (pH 8.0) containing glucose (2%) in a 500 ml shaking flask. The mixture was shaken at 200 rpm and 30°C and the bioconversion was followed by analytical HPLC. The reaction was stopped at 2 h with 96% conversion to *N-tert*-butoxycarbonyl 4-hydroxypiperidine. Standard work-up and column chromatography on silica gel (R_{f} = 0.20, *n*-hexanelethyl acetate 1:1) gave 69.3 mg (69.5%) of *N-tert*-butoxycarbonyl 4-hydroxypiperidine.
¹H-NMR (CDCl₃): δ 3.87-3.78 (*m*, 3 H, H_{A}-C(2), H_{A}-C(6), H-C(4)), 3.07-2.98 (*ddd*, 2 H, *J=* 13.5, 9.7, and 3.4 Hz, H_{B}-C(2), H_{B}-C(6)), 2.03 (*s*, 1 H, OH), 1.89-1.66 *(m,* 2 H, H_{A}-C(3), H_{A}-C(5)), 1.52-1.38 ppm *(m,* 11 H, H_{B}-C(3), H_{B}-C(5), and 3CH₃).
¹³C-NMR (CDCl₃): δ 154.86 (*s*, CO); 79.57 (*s*, OC(CH₃)₃); 67.69 (*d*, C-4); 41.26(*t*, C-2, C-6); 34.17 (*t*, C-3, C-5); 28.44 ppm (*q*, CH₃).
MS (80eV): m/e 202 (7%, M+1), 146 (24%), 102 (100%).

### Example 7: Preparation of N-benzoyl 4-hydroxypiperidine by hydroxylation of N-benzoyl piperidine with resting cells of Sphingomonas sp. HXN-200

*N*-Benzoyl piperidine (37.8 mg, 0.20 mmol) was added to 100 ml of cell suspension (4.0 g/L) of *Sphingomonas sp.* HXN-200 in 50 mM K-phosphate buffer (pH 8.0) containing glucose (2%) in a 500 ml shaking flask. The mixture was shaken at 200 rpm and 30°C and the bioconversion was followed by analytical HPLC. The reaction was stopped at 5 h with 83% conversion to *N*-benzoyl 4-hydroxypiperidine. Standard work-up and column chromatography on silica gel (R_{f} = 0.14, ethyl acetate) gave 29.3 mg (71.5%) of *N*-benzoyl 4-hydroxypiperidine.
¹H-NMR (CDCl₃): δ 7.45-7.36 (*m*, 5 H, aromatic H), 4.22 (*m*, 1 H, H_{A}-C(2 or 6)), 3.92 (*s*, 1 H, H-C(4)), 3.66 *(dt,* 1 H, J = 13.8, 4.5 Hz, H_{A}-C(6 or 2)), 3.32 *(t,* 1 H, *J* = 9.6, H_{B}-C(2 or 6)), 3.16 *(ddd,* 1H, *J* = 13.7, 9.3, and 3.3 Hz, H_{B}-C(6 or 2)), 2.90 (*s*, 1 H, OH), 1.95 (*m*, 1 H, H_{A}-C(3 or 5)), 1.80 (*m*, 1 H, H_{A}-C(5 or 3)), 1.60 (*ddt*, 1 H, *J*= 13.0, 9.0, and 4.0 Hz, H_{B}-C(3 or 5)), 1.46 ppm (*ddt*, 1 H, *J*= 12.7, 9.0, and 3.9Hz, H_{B}-C(3)).
¹³C-NMR (CDCl₃): δ 170.62 (*s*, CO); 135.70 (*s*), 129.91 (*d*), 128.71 (*d*), 126.80 (*d*) (aromatic C); 66.83 (*d*, C-4); 45.20 (*t*), 39.62 (*t*), (C-2, C-6); 34.42 (*t*,), 33.76 ppm (*t*), (C-2, C-5).
MS (80eV): m/e 206 (100%, M+1).

### Example 8: Preparation of N-benzoyl 4-hydroxypiperidine by hydroxylation of N-benzoyl piperidine with resting cells of Sphingomonas sp. HXN-200

*N*-Benzoyl piperidine (56.7 mg, 0.30 mmol) was added to 100 ml of cell suspension (4.0 g/L) of *Sphingomonas sp.* HXN-200 in 50 mM K-phosphate buffer (pH 8.0) containing glucose (2%) in a 500 ml shaking flask. The mixture was shaken at 200 rpm and 30°C and the bioconversion was followed by analytical HPLC. The reaction was stopped at 5 h with 62% conversion to *N*-benzoyl 4-hydroxypiperidine. Standard work-up and column chromatography on silica gel gave 32.1 mg (52.2%) of *N*-benzoyl 4-hydroxypiperidine.

### Example 9: Preparation of N-benzyl 4-hydroxypiperidine by hydroxylation of N-benzyl piperidine with resting cells (4.0 g/L) of HXN-200 in 2 L scale

*N*-Benzyl piperidine (1.75 g, 10 mmol) was added to a cell suspension (4.0 g/L) of *Sphingomonas sp.* HXN-200 in 2 L of 50 mM of K-phosphate buffer (pH 8.0) containing glucose (2%, w/v) in a 3 L bioreactor, the mixture was stirred at 1500 rpm and at 30°C under the introduction of air at 1 L/min. The biotransformation was stopped at 4 h with nearly 100% conversion to *N*-benzyl 4-hydroxypiperidine. Standard work-up and column chromatography on silica gel (R_{f} *=* 0.2, ethyl acetate/MeOH 8:2) gave 1.50 g (83%) of pure product as white powder. White crystals were obtained by crystallization from ethyl acetate/hexane (1/3).
¹H-NMR (CDCl₃): δ 7.31 (*s*, 2 H, aromatic H), 7.30 (*s*, 2 H, aromatic H), 7.29-7.21 (*m*, 1 H, aromatic H), 3.68 (*m*, 1 H, H-C(4)), 3.50 (*s*, 2 H, PhCH₂), 2.75 (*dt*, 2 H, *J* = 11.6, 4.0 Hz, H_{A}-C(2), H_{A}-C(6)), 2.14 *(dt,* 2 H, *J* = 12.2, 2.8 Hz, H_{B}-C(2), H_{B}-C(6)), 1.91-1.82 (*m*, 2 H, H_{A}-C(3), H_{A}-C(5)), 1.78 (*s*, *br*., 1 H, OH), 1.64-1.52 ppm (m, 2 H, H_{B}-C(3), H_{B}-C(5)).
¹³C-NMR (CDCl₃): δ 138.41 (*s*), 129.08 (*d*), 128.15 (*d*), 126.95 (*d*) (aromatic C); 68.07 (*d*, C-4); 62.92 (*t,* PhCH₂); 50.99 *(t,* C-(2), C-(6)); 34.49 *(t,* C-(3), C-(5)).
MS (80eV): m/e 192 (100%, M+1).

### Example 10: Preparation of N-benzyl-4-hydroxypiperidine by hydroxylation of N-benzyl piperidine with resting cells (10.2 g) of Sphingomonas sp. HXN-200 in 1 L scale

*N*-Benzyl piperidine (2.63 g, 15 mmol) was added to a cell suspension (10.2 g/L) of *Sphingomonas sp.* HXN-200 in 1 L of 50 mM of K-phosphate buffer (pH 8.0) containing glucose (2%, w/v) in a 3 L bioreactor, the mixture was stirred at 1500 rpm and at 30°C under the introduction of air at 2 L/min. The biotransformation was stopped at 5.2 h with 98% conversion to *N*-benzyl 4-hydroxypiperidine. Standard work-up afforded crude product (98% purity) which was subjected to column chromatography on aluminium oxide with ethyl acetate/hexane (1:1) and methanol/ethyl acetate (20/80). This gave 2.07 g (72%) of pure *N*-benzyl 4-hydroxypiperidine as white powder.

### Example 11: Preparation of N-benzyl 4-hydroxypiperidine by hydroxylation of N-benzyl piperidine with growing cells of Sphingomonas sp. HXN-200 in 1 L scale

The cells of *Sphingomonas sp.* HXN-200 were grown in 1 L E2 medium first on glucose and then on *n*-octane to a cell density of 6.2 g/L. *N*-benzyl piperidine (0.875 g, 5 mmol) was added, and the mixture was stirred at 1536 rpm and at 30°C with air introduction at 2 L/min. *n*-Octane vapour was still introduced during bioconversion and the cells were grown. Additional substrate was added at 30 min. (0.875 g, 5 mmol), 60 min. (0.875 g, 5 mmol), and 90 min. (0.875 g, 5 mmol). The reaction was followed by analytical HPLC and stopped at 2 h with 85% conversion to *N*-benzyl 4-hydroxypiperidine. Standard work-up and column chromatography on aluminium oxide with ethyl acetate/hexane (1:1) and methanol/ethyl acetate (20/80) afforded 2.866 g (75%) of pure product.

### Example 12: Preparation of N-benzyl-4-hydroxypiperidine by hydroxylation of N-benzyl piperidine with cell-free extract of Sphingomonas sp. HXN-200

Cells of HXN-200 were suspended in 10 ml of Tris-HCl buffer (pH = 7.5) to a density of 20 g/L. After passage through the French press three times, the cell debris was removed by centrifugation at 45000 rpm (Rotor Type 50.2 Ti) for 45 min. yielding soluble cell-free extracts containing no membrane proteins. To this cell-free extracts was added NADH (100 µl of 500 mM aqueous solution, 0.05 mmol) and *N*-benzyl piperidine (8.8 mg, 0.05 mmol). The mixture was shaken at 200 rpm and at 30°C for 1 h afforded 90% of *N*-benzyl-4-hydroxypiperidine.

### Example 13: Preparation of N-benzyl-4-hydroxypiperidine by hydroxylation of N-benzyl piperidine with resting cells of cyclohexane-degrading strain LD-5

Cyclohexane-degrading strain LD-5 (isolated by Li, Z. and Deutz, W., ETH Zurich; in the strain collection of Institute of Biotechnology, ETH Zurich) was inoculated in 1/4 of Evans medium without carbon source and grown on vapour of cyclohexane diluted 10 times by air at r.t. for 3 days. The cells were harvested and resuspened to 5 g/L in 50 mM K-phosphate buffer (pH 7.2) containing glucose (2% w/v). *N*-benzyl piperidine was added to a concentration of 5 mM, and the mixture was shaken at 30°C for 2 h. 60% of *N*-benzyl-4-hydroxypiperidine was obtained.

## Claims

1. A process for the preparation of *N*-substituted 4-hydroxypiperidine, wherein an oxygen atom is inserted regioselectively into the corresponding *N*-substituted piperidine, by using, as a biocatalyst, a bacterium degrading alkanes or alicyclic hydrocarbons, or a prokaryotic host-organism having the gene(s) necessary for the hydroxylation derived from the said bacterium, or an enzyme having hydroxylation activity derived therefrom.

2. The process of claim 1, wherein the bacterium is selected from the group consisting of bacteria degrading *n*-alkane containing 4 to 20 carbon atoms.

3. The process of claim 2, wherein the bacterium is selected from the group consisting of bacteria degrading *n*-octane.

4. The process of claim 3, wherein the bacterium is selected from the group consisting of the isolates *Sphingomonas sp.* HXN-200, HXN-100, HXN-1400, HXN-1500, PN3, PN21, PN26, PN27, PN32, S69, S70, *Pseudomonas putida* P1, and *Pseudomonas oleovorans* GPo1 (ATCC 29347).

5. The process of claim 2, wherein the bacterium is selected from the group consisting of bacteria degrading *n*-hexane.

6. The process of claim 2, wherein the bacterium is selected from the group consisting of bacteria degrading *n*-decane.

7. The process of claim 2, wherein the bacterium is selected from the group consisting of bacteria degrading *n*-dodecane.

8. The process of claim 2, wherein the bacterium is selected from the group consisting of bacteria degrading *n*-tetradecane.

9. The process of claim 1, wherein the bacterium is selected from the group consisting of bacteria degrading mono-alicyclic compounds containing 4 to 20 carbon atoms.

10. The process of claim 9, wherein the bacterium is selected from the group consisting of bacteria degrading cyclohexane.

11. The process of claim 10, wherein the bacterium is cyclohexane-degrading strain LD-5.

12. The process of claim 9, wherein the bacterium is selected from the group consisting of bacteria degrading cyclopentane.

13. The process of claim 9, wherein the bacterium is selected from the group consisting of bacteria degrading cycloheptane.

14. The process of claim 9, wherein the bacterium is selected from the group consisting of bacteria degrading cyclooctane.

15. The process of claim 1, wherein the biocatalyst is a recombinant bacterium carrying gene(s) necessary for the hydroxylation derived from a bacterium degrading alkanes or alicyclic hydrocarbons.

16. The process of claim 15, wherein the biocatalyst is a recombinant *Escherichia coli.* strain.

17. The process of claim 16, wherein the biocatalyst is *Escherichia coli* GEc137(pGEc47).

18. The process of claim 1, wherein resting bacterial cells, growing bacterial cells, or both, are used as biocatalyst.

19. The process of claim 1, wherein a crude cell extract, or a purified, or partially purified, enzyme preparation is used as biocatalysts.

20. The process of claim 1, wherein the biocatalyst is immobilized on or in a water-insoluble carrier or support system.

21. The process of claim 1, wherein the biocatalytic reaction is performed in aqueous medium.

22. The process of claim 1, wherein the biocatalytic reaction is performed in multiphase media containing two or more of the following: a solid phase, an aqueous phase, an organic phase, and a gaseous phase.

23. The process of claim 22, wherein organic phase is used which comprises one or more alkanes with 5 or more C atoms, dialkyl ethers with 4 or more C atoms, carboxylic esters with 4 or more C atoms, or aromatic or heteroaromatic hydrocarbons, optionally with substitution.

24. The process of claim 1, wherein the reaction temperature is 5-50°C, preferably 20-40°C.

25. The process of claim 1, wherein the pH of the medium is 4-10, preferably 6-8.

26. The process of claim 1, wherein the product is separated by column chromatography with an inorganic, organic or synthetic adsorbent used as a support.

27. The process of claim 1, wherein the product is separated by means of extraction, wherein the substrate is first recovered from the reaction mixture by extraction with less polar solvent, the remaining reaction mixture is adjusted to pH =10-12, and the product is extracted out with more polar solvent.

28. The process of claim 27, wherein the extraction agent used is selected from the group consisting of alkanes with 5 or more C atoms, dialkyl ethers with 4 or more C atoms, chlorine-containing alkanes with 3 or fewer C atoms, alkyl aromatics with 7-10 C atoms, and carboxylic esters with 3 or more C atoms.

29. The process of claim 1, wherein the product is separated by use of membrane filtration.

30. The process of claim 1, wherein the *N*-substituted 4-hydroxypyrrolidine is *N*-benzyl 4-hydroxypyrrolidine.

31. The process of claim 1, wherein the *N*-substituted 4-hydroxypyrrolidine is *N*-benzyloxycarbonyl 4-hydroxypyrrolidine.

32. The process of claim 1, wherein the *N*-substituted 4-hydroxypyrrolidine is *N*-phenoxycarbonyl 4-hydroxypyrrolidine.

33. The process of claim 1, wherein the *N*-substituted 4-hydroxypyrrolidine is *N-tert*-butoxycarbonyl 4-hydroxypyrrolidine.

34. The process of claim 1, wherein the *N*-substituted 4-hydroxypyrrolidine is *N*-benzoyl 4-hydroxypyrrolidine.
